(19) **Europäisches Patentamt** / **European Patent Office** / **Office européen des brevets**

(11) **EP 3 749 210 B1**

(12) **EUROPEAN PATENT SPECIFICATION**

(45) Date of publication and mention of the grant of the patent:
**29.11.2023 Bulletin 2023/48**

(21) Application number: **19706252.4**

(22) Date of filing: **09.02.2019**

(51) International Patent Classification (IPC):
**A61B 8/08** (2006.01)    **G01S 7/52** (2006.01)

(52) Cooperative Patent Classification (CPC):
**A61B 8/485; A61B 8/08; G01S 7/52022; G01S 7/52042; G01S 7/52074; G01S 7/52084;** A61B 8/4405; A61B 8/4427; A61B 8/465; A61B 8/467; A61B 8/469; A61B 8/483; A61B 8/5253; G01S 15/8995

(86) International application number:
**PCT/EP2019/053218**

(87) International publication number:
**WO 2019/155037 (15.08.2019 Gazette 2019/33)**

(54) **MULTI-PARAMETRIC TISSUE STIFFNESS QUANTIFICATION**

MULTIPARAMETRISCHE GEWEBESTEIFIGKEITSQUANTIFIZIERUNG

QUANTIFICATION DE RIGIDITÉ DE TISSU MULTI-PARAMÉTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2018 US 201862628323 P**

(43) Date of publication of application:
**16.12.2020 Bulletin 2020/51**

(73) Proprietor: **Koninklijke Philips N.V.**
**5656 AG Eindhoven (NL)**

(72) Inventors:
- **XIE, Hua**
  **5656 AE Eindhoven (NL)**
- **NGUYEN, Man**
  **5656 AE Eindhoven (NL)**
- **HUANG, Sheng-Wen**
  **5656 AE Eindhoven (NL)**
- **AMADOR CARRASCAL, Carolina**
  **5656 AE Eindhoven (NL)**
- **SHAMDASANI, Vijay, Thakur**
  **5656 AE Eindhoven (NL)**
- **ROBERT, Jean-Luc, Francois-Marie**
  **5656 AE Eindhoven (NL)**

(74) Representative: **Philips Intellectual Property & Standards**
**High Tech Campus 52**
**5656 AG Eindhoven (NL)**

(56) References cited:
**WO-A1-2017/197404    US-A1- 2018 000 455**

- **GENNISSON J L ET AL: "Viscoelastic and Anisotropic Mechanical Properties of in vivo Muscle Tissue Assessed by Supersonic Shear Imaging", ULTRASOUND IN MEDICINE AND BIOLOGY, NEW YORK, NY, US, vol. 36, no. 5, 1 May 2010 (2010-05-01), pages 789-801, XP027029726, ISSN: 0301-5629 [retrieved on 2010-04-24]**
- **WEI-NING LEE ET AL: "Mapping Myocardial Fiber Orientation Using Echocardiography-Based Shear Wave Imaging", IEEE TRANSACTIONS ON MEDICAL IMAGING, IEEE SERVICE CENTER, PISCATAWAY, NJ, US, vol. 31, no. 3, 1 March 2012 (2012-03-01), pages 554-562, XP011491045, ISSN: 0278-0062, DOI: 10.1109/TMI.2011.2172690**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- WANG MICHAEL ET AL: "Imaging Transverse Isotropic Properties of Muscle by Monitoring Acoustic Radiation Force Induced Shear Waves Using a 2-D Matrix Ultrasound Array", IEEE TRANSACTIONS ON MEDICAL IMAGING, IEEE SERVICE CENTER, PISCATAWAY, NJ, US, vol. 32, no. 9, 1 September 2013 (2013-09-01), pages 1671-1684, XP011525111, ISSN: 0278-0062, DOI: 10.1109/TMI.2013.2262948 [retrieved on 2013-08-28]
- SÉBASTIEN AUBRY ET AL: "Viscoelasticity in Achilles Tendonopathy: Quantitative Assessment by Using Real-time Shear-Wave Elastography", RADIOLOGY, vol. 274, no. 3, 1 March 2015 (2015-03-01) , pages 821-829, XP055597243, US ISSN: 0033-8419, DOI: 10.1148/radiol.14140434
- LI YIN ET AL: "Three-Dimensional Shear Wave Elastography of Skeletal Muscle: Preliminary Study : 3D Shear Wave Elastography of Skeletal Muscle", JOURNAL OF ULTRASOUND IN MEDICINE., vol. 37, no. 8, 5 February 2018 (2018-02-05), pages 2053-2062, XP055597146, United States ISSN: 0278-4297, DOI: 10.1002/jum.14559

## Description

TECHNICAL FIELD

The present disclosure pertains to ultrasound systems and methods for determining stiffness levels of anisotropic tissues using shear wave elastography.

BACKGROUND

Ultrasound shear wave elastography has been used to measure localized stiffness levels of various tissues, which may provide valuable information for detecting tissue abnormalities and diagnosing conditions such as cancer or liver fibrosis. Ultrasound shear wave elastography typically involves transmitting a "push pulse" from a transducer into a tissue, thereby generating a shear wave that propagates laterally therethrough. Tracking pulses emitted by the transducer can then be used to measure the velocity of the shear wave as it propagates, which usually fluctuates based on the stiffness of the tissue. For example, shear wave velocity in soft tissue is typically slower than shear wave velocity in hard tissue, assuming an identical push pulse is used to generate the shear wave in each tissue type. Accordingly, variation in shear wave velocity can be used to distinguish normal, soft tissues from abnormal, hard tissues.

While preexisting ultrasound elastography systems have proven effective in measuring localized tissue stiffness levels in organs like the liver, breast, prostate and thyroid, the tissues comprising such organs are primarily isotropic. As such, the stiffness levels of the tissues are approximately identical in all directions. The stiffness levels of muscle tissue and tissue comprising the tendons, kidneys and the heart exhibit anisotropic mechanical properties, i.e., tissue stiffness levels that differ in different directions. Stiffness quantification of such tissues is hampered by a lack of understanding regarding complex shear wave propagation dependence on the ultrasound system and material structural characteristics, and the unavailability of imaging modalities capable of fully characterizing anisotropic tissue properties. New ultrasound systems configured to determine the stiffness levels of anisotropic tissues via shear wave elastography are therefore needed.

Document "Viscoelastic and Anisotropic Mechanical Properties of in vivo Muscle Tissue Assessed by Supersonic Shear Imaging" by J. L. Gennisson et al., Ultrasound in Medicine and Biology, New York, NY, US, vol. 36, no. 5, 1 May 2010, pp. 789-801, XP027029726, ISSN: 0301-5629, discloses a method for performing local elasticity measurements of an anisotropic tissue using shear wave ultrasound measurements.

Document "Mapping Myocardial Fiber Orientation Using Echocardiography-Based Shear Wave Imaging" by W.-N. Lee et al., IEEE Transactions on Medical Imaging, IEEE Service Center, Piscataway, NJ, US, vol. 31, no. 3, 1 March 2012, pp. 554-562, XP011491045, ISSN: 0278-0062, DOI: 10.1109/ TMI.2011.2172690, discloses a method for characterising soft tissue using shear waves generated by ultrasound.

Document US 2018/000455 A1 discloses a method for performing shear wave ultrasound imaging based on spatial characteristics of an anisotropic medium being imaged.

Document "Imaging Transverse Isotropic Properties of Muscle by Monitoring Acoustic Radiation Force Induced Shear Waves Using a 2-D Matrix Ultrasound Array" by M. Wang et al., IEEE Transactions on Medical Imaging, IEEE Service Center, Piscataway, NJ, US, vol. 32, no. 9, 1 September 2013, pp. 1671-1684, XP011525111, ISSN: 0278-0062, DOI: 10.1109/TMI.2013.2262948, discloses a method of monitoring shear wave propagation in muscle.

Document "Viscoelasti city in Achilles Tendonopathy: Quantitative Assessment by Using Real-time Shear-Wave Elastography" by S. Aubry et al., Radiology, vol. 274, no. 3, 1 March 2015, pp. 821-829, XP055597243, US ISSN: 0033-8419, DOI: 10.1148/radiol.14140434, discloses a method of investigating the viscoelastic properties of a tendon using shear-wave elastography.

SUMMARY

The present disclosure describes systems and methods for determining stiffness levels of anisotropic tissue via shear wave ultrasound imaging. The anisotropic tissue, e.g., skeletal muscle, evaluated according to the methods described herein may be angled with respect to a nominal axial direction of the ultrasound transducer used to interrogate the tissue. To accurately track the propagation pattern of the shear wave generated from push pulses transmitted into the tissue, systems herein are configured to determine the angular orientation of the tissue and adjust the steering angle of the push pulses based on the determined orientation. Push pulses can be emitted at various steering angles to fully characterize the tissue, while tracking pulses arranged parallel to the push pulses monitor tissue displacement caused by the resulting shear waves. Displacement data acquired by the system can then be used to determine the velocity of the shear waves at various points within the tissue. The velocity data is indicative of the tissue stiffness. The system can also reconstruct all three mechanical moduli (i.e. longitudinal shear modulus, transverse shear modulus and longitudinal Young's modulus) based on multi-angle shear wave speed measurement for full characterization of the anisotropic tissue under investigation if it is transversely isotropic material (skeletal muscle is often considered transversely isotropic).

In accordance with principles of the present disclosure, an ultrasound imaging system for shear wave imaging is provided in claim 1.

In some embodiments, the processor is configured to determine the angular orientation responsive to user input. In some examples, the processor is further configured to automatically determine the angular orientation of the target tissue based on the acquired echoes. In some embodiments, the processor is configured to determine the angular orientation of the target tissue by determining the maximum intensity of backscattering signals generated at a plurality of image beam steering angles transmitted by the beamformer after compensating the beam pattern directivity. In some examples, the processor is configured to determine an angular orientation of the target tissue by performing a Hough transform on image frames generated from the acquired echoes. In some embodiments, the processor is configured to detect motion by determining tissue displacement in a lateral and an axial direction caused by the shear wave. In some examples, the processor is configured to measure the velocity of the shear wave in the lateral and the axial direction. In some embodiments, the processor is further configured to generate a shear wave map based on the measured velocity of the shear wave, the shear wave map comprising a display of a two dimensional image of shear wave velocity values. In some embodiments, the processor is further configured to determine multiple shear wave velocities obtained at a plurality of push pulse steering angles and angular tissue orientations. In some embodiments, the processor is further configured to determine multi-parametric stiffness values of the target tissue based on the multiple shear wave velocities. In some examples, the beamformer is configured to transmit, from the ultrasound transducer, a plurality of push pulses, each push pulse transmitted at a distinct steering angle with respect to the target tissue such that a first push pulse is transmitted parallel to the target tissue, a second push pulse is transmitted perpendicular to the target tissue, and a third push pulse is transmitted at an oblique angle with respect to the target tissue.

A method of shear wave imaging in accordance with the present disclosure is provided in claim 12.

In some examples, the method may involve determining the angular orientation of the target tissue. In some embodiments, determining the angular orientation of the target tissue comprises determining an intensity of backscattering signals generated at a plurality of image beam steering angles transmitted by the beamformer. In some examples, detecting motion within the target tissue comprises determining tissue displacement in a lateral and an axial direction caused by the shear wave. Example methods may further involve measuring the velocity of the shear wave in the lateral and the axial direction. In some embodiments, transmitting a push pulse comprises transmitting a plurality of push pulses, each push pulse transmitted at a distinct steering angle with respect to the target tissue such that a first push pulse is transmitted parallel to the target tissue, a second push pulse is transmitted perpendicular to the target tissue, and a third push pulse is transmitted at an oblique angle with respect to the target tissue. Embodiments may also involve determining multiple shear wave velocities obtained at a plurality of push pulse steering angles and angular tissue orientations.

Any of the methods described herein, or steps thereof, may be embodied in non-transitory computer-readable medium comprising executable instructions, which when executed may cause a processor of a medical imaging system to perform the method or steps embodied herein.

Advantageous embodiments are defined in the dependent claims.

## BRIEF DESCRIPTION OF THE DRAWINGS

FIG. 1A is an ultrasound image of biceps brachii muscle.
FIG. 1B is a diagram of the ultrasound probe placement used to acquire the image of FIG. 1A.
FIG. 1C is an ultrasound image of medial gastrocnemius muscle.
FIG. 1D is a diagram of the ultrasound transducer placement used to acquire the image of FIG. 1C.
FIG. 2 is a block diagram of an ultrasonic imaging system constructed in accordance with the principles of the present invention.
FIG. 3A is an example display screen showing a live ultrasound image of anisotropic tissue at a first angular orientation.
FIG. 3B is an example display screen showing a live ultrasound image of the anisotropic tissue shown in FIG. 3A at a second angular orientation.
FIG. 3C is an example display screen showing a live ultrasound image of the anisotropic tissue shown in FIG. 3A at a third angular orientation.
FIG. 4 is an example display screen showing a live ultrasound image and a list view of a custom shear wave imaging technique.
FIG. 5A is an example display screen showing a live ultrasound image and an image-based view of a custom shear wave imaging tech
FIG. 5B is an example of a user interface that can be used to generate the live ultrasound image of FIG. 5A.
FIG. 6 shows nine ultrasound images of a target tissue arranged parallel to the lateral direction of an ultrasound transducer imaging plane, each image obtained at a distinct steering angle.
FIG. 7 shows nine ultrasound images of a target tissue arranged at an oblique angle with respect to the lateral direction of an ultrasound transducer imaging plane, each image obtained at a distinct steering angle.
FIG. 8A is a compounded image generated from the images shown in FIG. 6.
FIG. 8B is a compounded image generated from the images shown in FIG. 7.
FIG. 8C is a compounded image generated by combining a plurality of images arranged at another oblique angle with respect to the lateral direction of an ultrasound transducer imaging plane.

FIG. 8D is a graphical representation of backscattering signal intensity measured as a function of beam steering angle for FIGS 8A-8C.

FIG. 9A is a stiffness map generated using conventionally-steered push/tracking beams.

FIG. 9B is a non-steered ROI tracking box displayed on an ultrasound image.

FIG. 9C is a steered ROI tracking box displayed on an ultrasound image.

FIG. 9D is a steered ROI tracking box with a different angle displayed on an ultrasound image.

FIG. 10 is an example report generated and displayed in accordance with principles of the present disclosure.

FIG. 11 is a method performed in accordance with principles of the present disclosure.

DETAILED DESCRIPTION

The following description of certain embodiments is merely exemplary in nature and is in no way intended to limit the invention or its applications or uses. In the following detailed description of embodiments of the present systems and methods, reference is made to the accompanying drawings which form a part hereof, and which are shown by way of illustration specific embodiments in which the described systems and methods may be practiced. These embodiments are described in sufficient detail to enable those skilled in the art to practice presently disclosed systems and methods, and it is to be understood that other embodiments may be utilized and that structural and logical changes may be made without departing from the spirit and scope of the present system. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

The present technology is also described below with reference to block diagrams and/or flowchart illustrations of methods, apparatus (systems) and/or computer program products according to the present embodiments. It is understood that blocks of the block diagrams and/or flowchart illustrations, and combinations of blocks in the block diagrams and/or flowchart illustrations, may be implemented by computer executable instructions. These computer executable instructions may be provided to a processor, controller or controlling unit of a general purpose computer, special purpose computer, and/or other programmable data processing apparatus to produce a machine, such that the instructions, which execute via the processor of the computer and/or other programmable data processing apparatus, create means for implementing the functions/acts specified in the block diagrams and/or flowchart block or blocks.

As described herein, anisotropic tissue refers to tissue exhibiting anisotropic mechanical properties, e.g., tissue stiffness levels that vary across different directions of the tissue fibers. For example, anisotropic tissue may be characterized by different stiffness values measured parallel to and perpendicular to the direction of the organized fibers comprising the tissue. Examples of anisotropic tissues contemplated herein include but are not limited to: muscle tissue, tendon tissue and kidney tissue. Subsets of anisotropic tissue contemplated herein include, for example, skeletal muscle tissue and myocardial tissue. For ease of description, the aforementioned tissue types will be referred to under the umbrella term "anisotropic."

Isotropic tissue refers to tissue exhibiting isotropic properties, e.g., tissue stiffness values that remain approximately constant across different directions of the tissue. Preexisting shear wave elastography systems have typically been programmed to assume that tissue comprising organs such as the liver, breast, prostate and thyroid are linear, incompressible and isotropic. As such, when determining the stiffness levels of such tissues, preexisting systems have only determined one physical parameter, i.e., Young's modulus, which can be expressed as a function of tissue density ($\rho$) and shear wave speed ($V_{sh}$), as shown in Equation 1.1:

$$[037] \quad \text{Equation 1.1.} \quad E = 3\rho V_{sh}^2$$

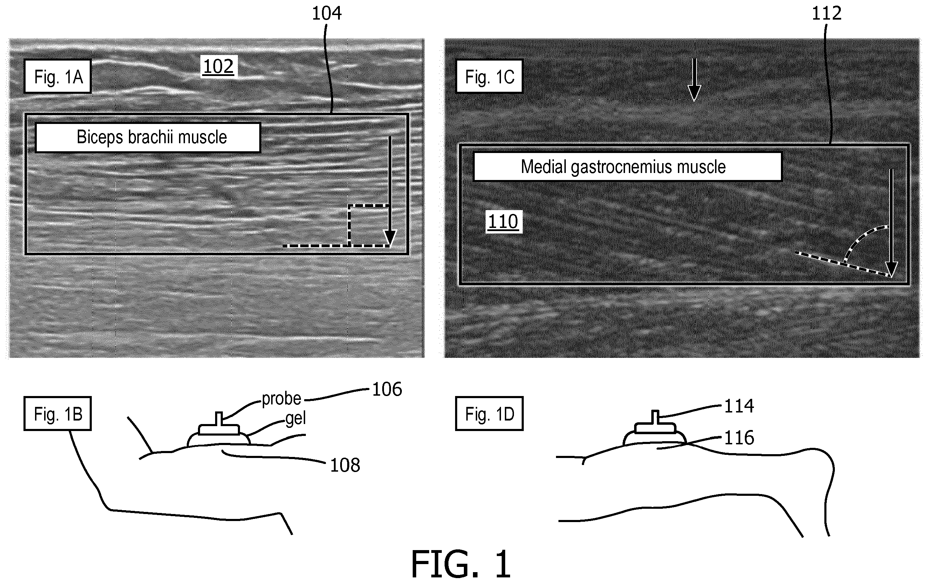

Young's modulus may be used to characterize the stiffness of isotropic tissue because the angle of the ultrasound transducer with respect to the tissue may not influence the propagation pattern of the shear wave produced upon transmitting a push pulse into the tissue. By contrast, adjusting the angle of the transducer when emitting push pulses into anisotropic tissue will change the propagation pattern of the shear wave through the tissue. For instance, FIGS. 1A and 1C show ultrasound images of anisotropic tissue comprised of two different types of skeletal muscle. As shown, the organized fibers of the two muscle types differ by the angle of the fibers with respect to the transducer used to image them. FIG. 1A is an ultrasound image of biceps brachii muscle fibers 102 and a shear wave tracking box 104. The fibers within the tracking box 104 are arranged naturally parallel to the lateral direction in the imaging plane of the ultrasound transducer 106 shown in FIG. 1B, which is placed over a patient's bicep 108. As a result, a push pulse transmitted into the fibers 102 (in the direction of the arrow) will pass through the fibers at an approximately perpendicular angle, generating a shear wave that propagates in the direction of the fibers. FIG. 1C is an ultrasound image of medial gastrocnemius muscle fibers 110 and a shear wave tracking box 112. In contrast to the fibers shown in FIG. 1A, the fibers within the tracking box 112 are arranged at an oblique angle to the lateral direction in the imaging plane of the transducer 114 shown in FIG. 1D, which is placed over a patient's calf 116. A push pulse transmitted into the fibers 110 (in the direction of the arrow) will pass through the fibers at an oblique angle, generating a shear wave that also propagates in the direction of the fibers. Due to the differently angled fibers in each muscle type, the shear wave propagation pattern generated by emitting a push pulse into each tissue will thus vary. As a result, the shear wave speed must be measured differently in each tissue type. Shear wave speed can be measured in the tissue of FIG. 1A by determining the longitudinal shear modulus, i.e., parallel to the fibers; however, shear wave speed can only be accurately measured in the tissue of FIG. 1C by identifying the transverse shear modulus, longitudinal shear modulus, and longitudinal Young's modulus, which requires measuring shear wave speed in three muscle fiber orientations relative to the transducer: (1) fiber in-plane and parallel to the lateral direction, (2) fiber cross-plane perpendicular to the imaging plane, and (3) fiber in-plane with a known tilt angle.

Provided herein are ultrasound-based shear wave elastography systems configured to determine tissue stiffness values in anisotropic tissues regardless of the angle of such tissues relative to the transducer. Systems described herein can be configured to determine the angular orientation of anisotropic tissue fibers and, based on the determined orientation, automatically conduct and/or guide a user to conduct customized shear wave elastography techniques, which can involve electronically steering push pulses and tracking beams at particular angles based on the determined fiber orientation. Embodiments can be further configured to perform shear wave tracking and wave speed reconstruction in a manner that is responsive to the determined fiber orientation and the parameters of the emitted ultrasound pulses. Shear wave speeds and stiffness values acquired under various acquisition conditions can be tabulated and displayed for full characterization of anisotropic tissues. By implementing the systems and methods described herein, the variability in anisotropic tissue stiffness measurements caused by different angulation can be reduced, thereby generating more accurate, standardized stiffness measurements in tissue exhibiting anisotropic mechanical properties. Applications of the technology described herein include detection of numerous tissue abnormalities including tumors, injuries, muscle weakness and/or fibrosis. Specific implementations may include detection of muscle diseases such as dystrophy and myositis. In some examples, the systems described herein may be used to determine muscle stiffness in different states, e.g., active contracting state vs. passive resting. In addition to various muscle types, anisotropic tissues can be examined in the kidney cortex.

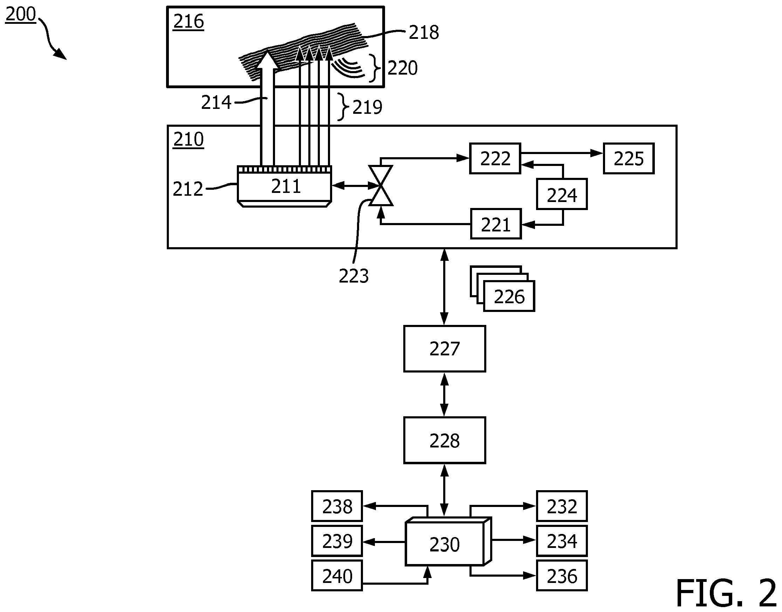

FIG. 2 shows an example ultrasound system 200 configured to perform shear wave elastography on anisotropic tissue in accordance with the present disclosure. As shown, the system 200 can include an ultrasound data acquisition unit 210, which can include an ultrasound probe 211 containing an ultrasound sensor array 212 configured to transmit and receive ultrasound signals. The array 212 is configured to emit an ultrasonic push pulse 214 into a target region 216 containing anisotropic tissue 218, e.g., musculoskeletal tissue. The array 212 is also configured to transmit a plurality of tracking pulses 219 into the anisotropic tissue 218 to detect shear wave propagation after transmission of the push pulse. The array 212 is coupled to a transmit beamformer 221 and a multiline receive beamformer 222 via a transmit/receive (T/R) switch 223. Coordination of transmission and reception by the beamformers 221, 222 can be controlled by a beamformer controller 224. The multiline receive beamformer 222 can produce spatially distinct receive lines (A-lines) of echo signals, which can be processed by filtering, noise reduction, etc. by a signal processor 225. In some embodiments, the components of the data acquisition unit 210 may be configured to generate a plurality of ultrasound image frames 226 from the ultrasound echoes 220 received at the array 212. The system 200 may also include one or more processors, such as a data processor 227, which may be configured to organize A-line data into groups and detect localized movement of the anisotropic tissue 218 based on the data embodied in each group of A-lines. Together, the components of the data acquisition unit 210 and the data processor 227 are configured to detect the velocity of a laterally or obliquely traveling shear wave by sensing and analyzing displacement of the anisotropic tissue 218 caused by the shear wave as it propagates through the tissue. Tracking displacement of the tissue may be achieved, in part, by time-interleaving the tracking pulses 219, for example as described in U.S. Application Publication No. 2013/0131511 Peterson et al.).

In various embodiments, the system 210 also includes a display processor 228 coupled with the data processor 227 and a user interface 230. The display processor 228 can be configured to generate ultrasound images 232 from the image frames 226, instructions 234 for performing shear wave elastography, a shear wave region of interest tracking box graphic 236 ("ROI tracking box"), one or more automated captions 238, and a live shear wave map 239, which may be based on the measured velocity of the shear wave and comprising a display of a two dimensional image of shear wave velocity values determined by the system 200. The user interface 230 can be configured to display the ultrasound images 232 in real time as an ultrasound scan is being performed, and may receive user input 240 at any time before, during or after a scan. The configuration of the system 200 shown in FIG. 2 may vary. For example, the system 200 can be portable or stationary. Various portable devices, e.g., laptops, tablets, smart phones, or the like, may be used to implement one or more functions of the system 200. In examples that incorporate such devices, the ultrasound sensor array 212 may be connectable via a USB interface, for example.

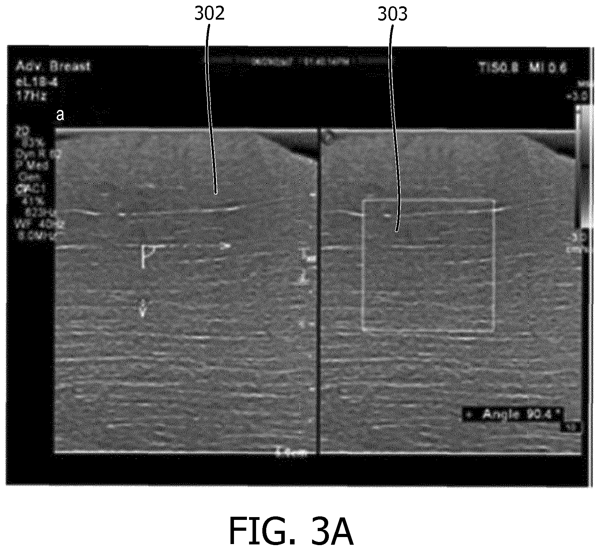

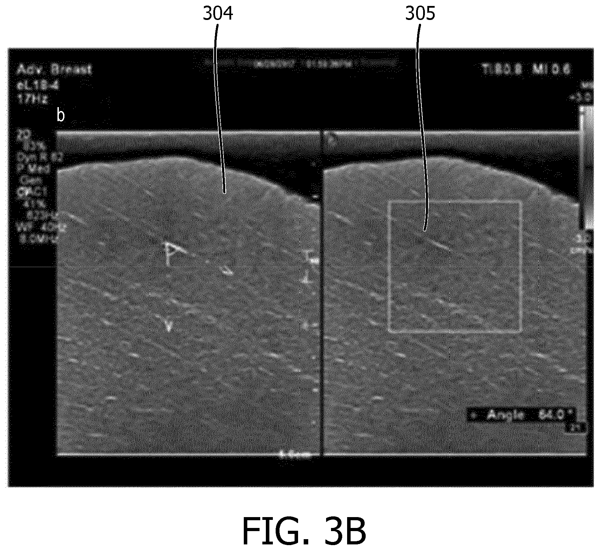

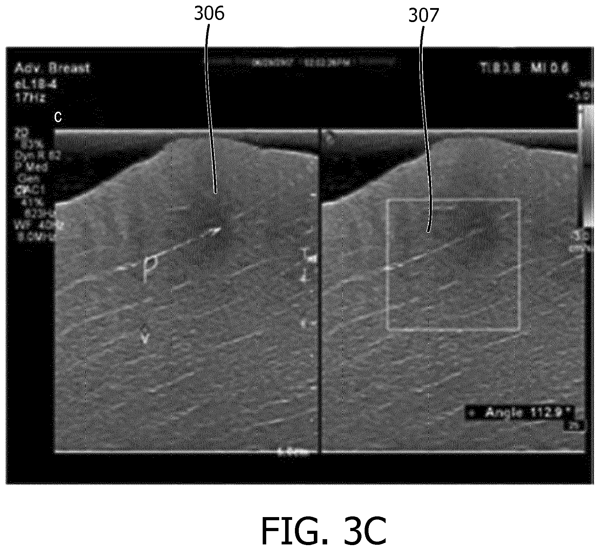

The system 200 can be configured to switch between multiple imaging and non-imaging modalities in response to receipt of the user input 240. One of the possible modalities includes shear wave imaging, which may contain separate sub-modalities for isotropic and anisotropic tissue elastography. In some examples, after the user instructs the system to enter the shear wave imaging mode for anisotropic tissue, a display screen configured to display a live ultrasound image may appear. An example of the display screen is provided in FIGS. 3A-3C, which show live ultrasound image displays of anisotropic tissue imaged from different transducer orientations with respect to the fiber orientation under a selectable in-plane setting. In some embodiments, the user can determine the angular orientation of the anisotropic tissue by visually inspecting the ultrasound images, e.g., B-mode images, of the tissue. In addition or alternatively, systems herein can be configured to automatically detect the fiber orientation and then electronically steer push/tracking pulses accordingly. The user may be presented with an option to measure fiber orientation via visual inspection, aided by a digital angle measurement tool, or through automated orientation measurement performed by the system.

Each of images 3A-3C shows tissue fibers arranged in a distinct angular orientation with respect to the ultrasound transducer used to image them. The fibers may be angled with respect to a nominal axial direction of the transducer, e.g., along the depth direction or generally perpendicular to the transducer. An angle measurement tool operating on the ultrasound system is configured to measure the angle of the fibers in each image 302, 304, 306 within a defined angle measurement box 303, 305, 307. In the particular example shown, the first image 302 shows tissue fibers oriented at approximately 90° with respect to the axial direction (or 0° with respect to the lateral direction), the second image 304 shows tissue fibers oriented at approximately 64° with respect to the axial direction (or 26° with respect to the lateral direction), and the third image 306 shows tissue fibers oriented at approximately 112.9° with respect to the axial direction (or -23° with respect to lateral direction).

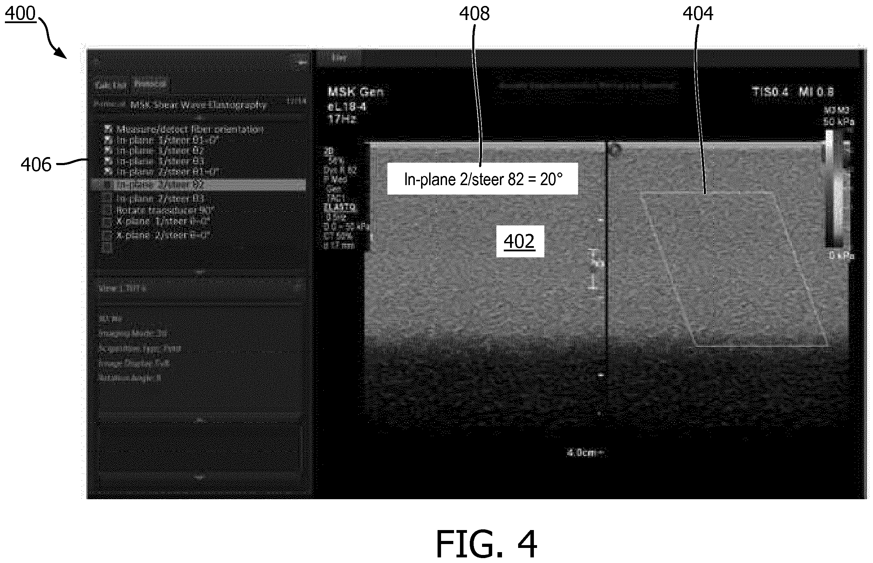

After determining the angular orientation of the tissue fibers, an ultrasound acquisition unit, e.g., unit 210, may be utilized to transmit push pulses and tracking pulses within the tissue at particular angles based on the angular orientation of the fiber, such that the resulting shear waves propagate through the tissue in a fiber in-plane and parallel to the lateral direction, a fiber cross-plane direction, and a fiber in-plane direction at a known tilt angle. In some examples, the system may be configured to guide the user through a customized sequence of push-pulse measurements to generate shear waves in these directions through the tissue. FIG. 4 illustrates an example display 400 that can be presented to the user on a user interface, e.g., user interface 230, to guide the user through a customized shear wave imaging protocol. As shown, the display 400 can include a live ultrasound image 402 of the targeted tissue and a shear wave ROI tracking box 404 overlaid thereon. The display 400 also includes a list of instructions 406, presented as a work- or checklist, for performing a shear wave scan sequence and acquiring shear wave speed measurements in the imaging plane and transverse to the imaging plane, i.e., cross-plane. In some examples, the display 400 can include a selectable icon for switching between the in-plane and cross-plane acquisition states. After determining the fiber orientation (which is the first instruction in the list of instructions 406), the system may update the instructions 406 to perform shear wave imaging at various steering angles. As shown, the instructions may be displayed in a checklist format, such that completion of each step is recorded in real time adjacent to each instruction. Sample instructions may include, for example, "Measure/detect fiber orientation"; "In-plane 1/steer $\theta1=0$°"; "Rotate transducer 90°"; or "X-plane 1/steer $\theta1=0$°." The default steering angle may be set at 0° in various embodiments.

The user may initiate shear wave imaging in compliance with the instructions 406 by adjusting the steering angle of the beamformer, e.g., transmit beamformer 221. Embodiments can include a rotary knob or digital control for adjusting the beam steering angle. In response to steering angle adjustment, the geometry of the shear wave ROI tracking box 404 may change in real-time. In the particular example shown in FIG. 4, a 20° beam steering angle has been specified by the user, as indicated by a caption 408 and as apparent by the tilted angle of the ROI tracking box 404. Under the direction of the user, parallel push/tracking pulses can be transmitted into the tissue at the specified 20° angle.

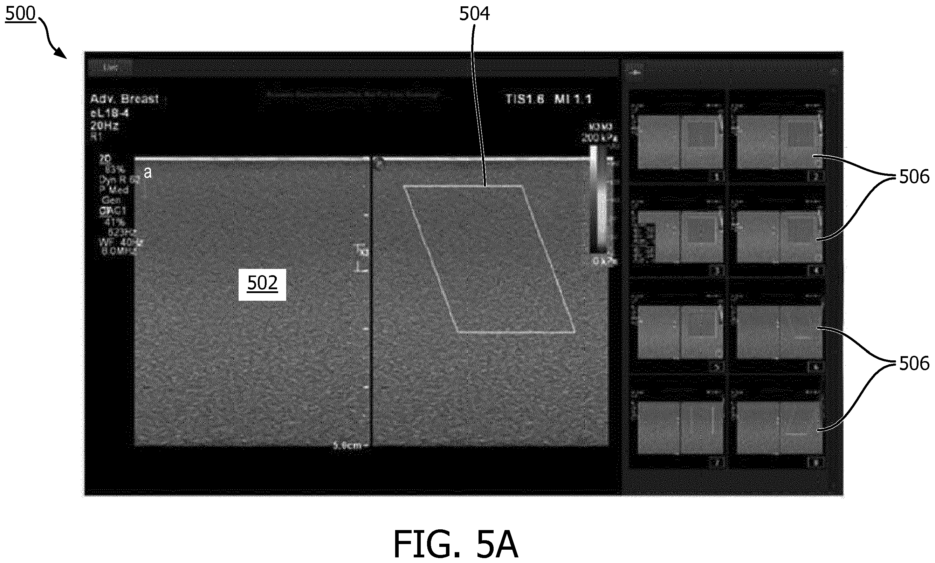

FIG. 5A shows another example of a display 500 that may be presented on a user interface. The display 500 includes a live ultrasound image 502 of the targeted tissue and an ROI tracking box 504 configured to change shape in response to beam steering adjustment input by a user. The display 500 also includes a plurality of angular beam steering status boxes 506. In the example shown, the status boxes 506 comprise thumbnail images of the targeted tissue with variously oriented ROI tracking boxes. Upon completion of shear wave tracking at a particular beam steering angle, the status box displaying the ROI tracking box modified to show the angle is filled in or shaded. The display 500 shown includes three unfilled status boxes 506, each status box representing a distinct beam steering angle to be transmitted in accordance with a customized shear wave protocol.

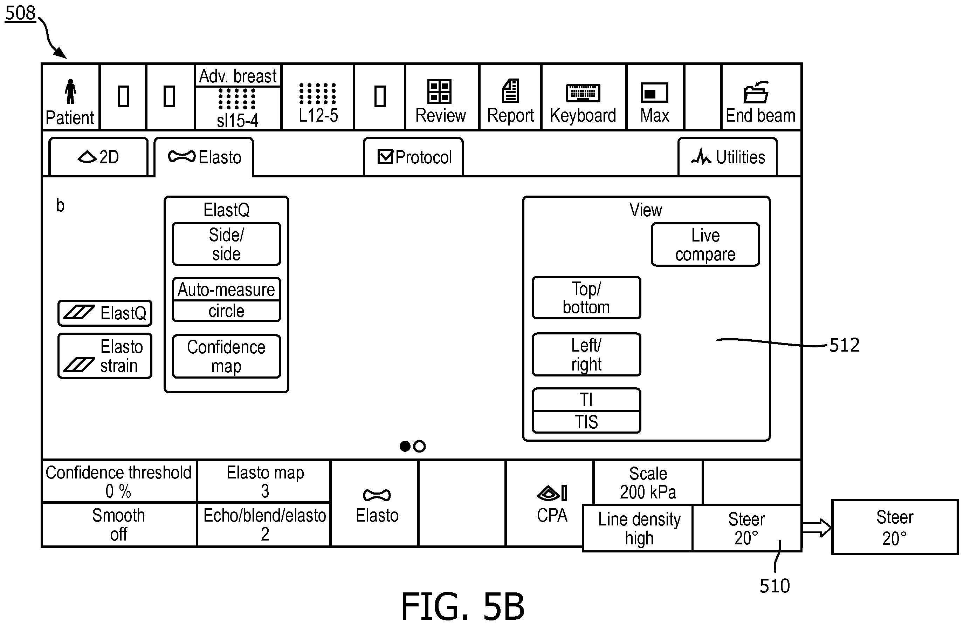

FIG. 5B shows an example of a user interface 508 used to generate the display 500 shown in FIG. 5A. As shown, a user may be presented with a plurality of interactive buttons for measuring fiber orientation and performing shear wave elastography responsive to the detected fiber orientation. Among other things, the user interface 508 includes a current beam steering angle 510, which in this particular example is 20°. The user interface 508 also displays a variety of viewing options 512, which include "Live Compare"; "Top/Bottom"; and "Left/Right."

As mentioned above, embodiments of the systems described herein may also be configured to automatically measure the fiber orientation of a targeted tissue and perform a customized sequence of push/tracking pulse transmission in response to the measured fiber orientation, without user input. Automated fiber orientation determination and push/tracking transmission may be implemented to reduce user interaction and eliminate measurement variability. Systems herein can be configured to determine fiber orientation in automated fashion according to multiple techniques, which may be implemented alone or in combination. For example, fiber orientation may be determined according to image-processing based methods, e.g., edge detection and Hough transform for linear feature detection, and/or acoustic-property based methods, which may involve steering ultrasound beams into the targeted tissue at various angles and then combining the resulting images together.

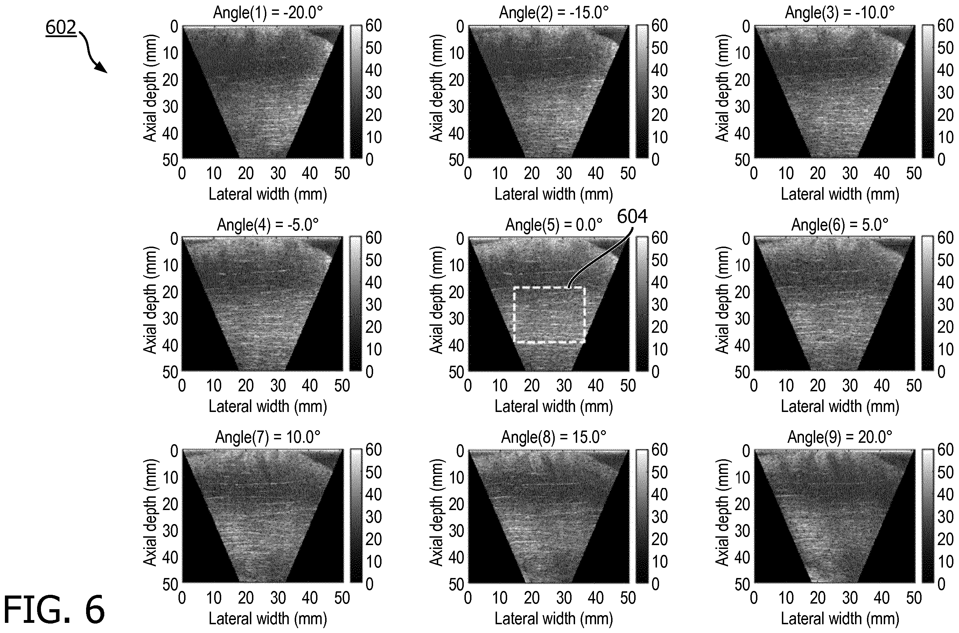

Systems configured to perform acoustic-property based methods to determine fiber orientation may be configured to implement SonoCT by Philips Koninklijke N.V. SonoCT is real-time compound imaging technology that can be employed by the systems herein to analyze ultrasound backscattering coefficient/strength. SonoCT may involve the transmitting ultrasound beams and receiving the corresponding ultrasound echoes at a variety of beam steering angles. From the received echoes, backscattering signal intensity within a region of interest may be determined at each angle, for example by determining the signal strength of each image on a pixel-by-pixel basis. The system can determine the peak intensity value and identify the tissue orientation angle that corresponds to the peak intensity value. An example of the compound imaging technology implemented by example systems herein is shown in FIGS. 6-8.

FIG. 6 shows nine B-mode ultrasound images obtained via nine distinct beam steering angles of anisotropic tissue arranged parallel to the lateral direction of the imaging plane. The beam steering angle used to acquire each image is indicated above each image. As shown, example beam steering angles may include -20°, -15°, -10°, -5°, 0°, 5°, 10°, 15°, and 20° with respect to the axial direction. At each angle, raw radio frequency data can be acquired via an ultrasound data acquisition unit, e.g., acquisition unit 210, and analyzed for backscattering intensity within a region of interest designated by the white box 604 shown in the middle image of FIG. 6. Of the nine images shown in FIG. 6, the observed backscattering intensity is the greatest in the image produced via a beam steering angle of 0° after taking into account the beam pattern directivity.

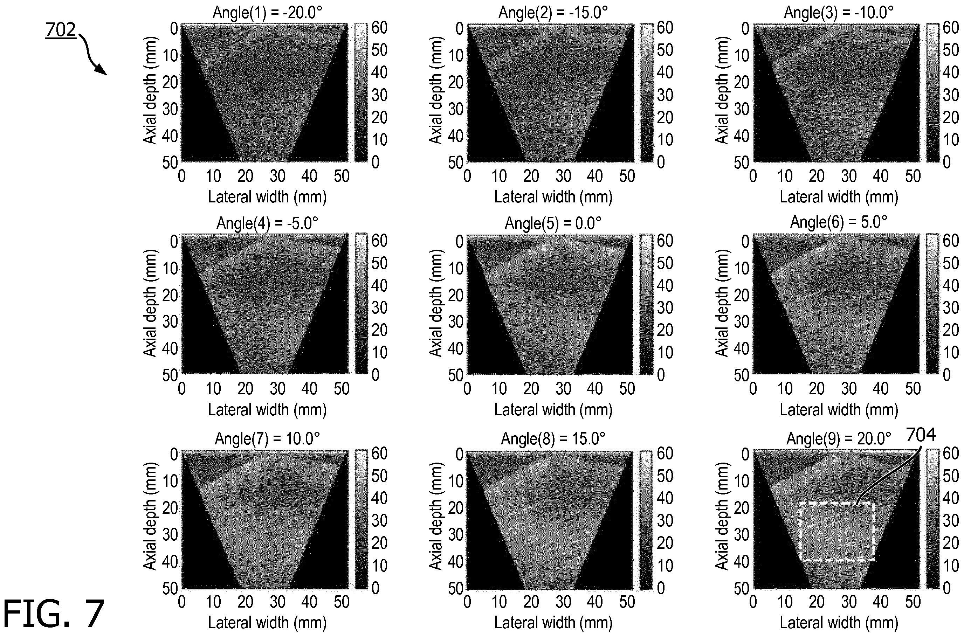

FIG. 7 shows nine B-mode ultrasound images 702 obtained via nine distinct beam steering angles of anisotropic tissue arranged at an oblique angle of approximately -23° with respect to the lateral direction of the imaging plane. The beam steering angle used to acquire each image is indicated above each image. As in FIG. 7, the example beam steering angles employed in this embodiment also include -20°, -15°, -10°, -5°, 0°, 5°, 10°, 15°, and 20°. The observed backscattering intensity is the greatest at the 20° beam steering angle, as shown by the white region of interest box 704.

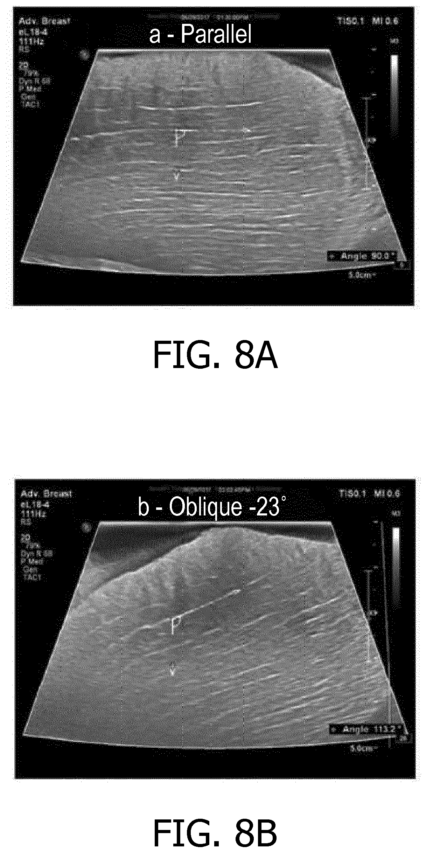

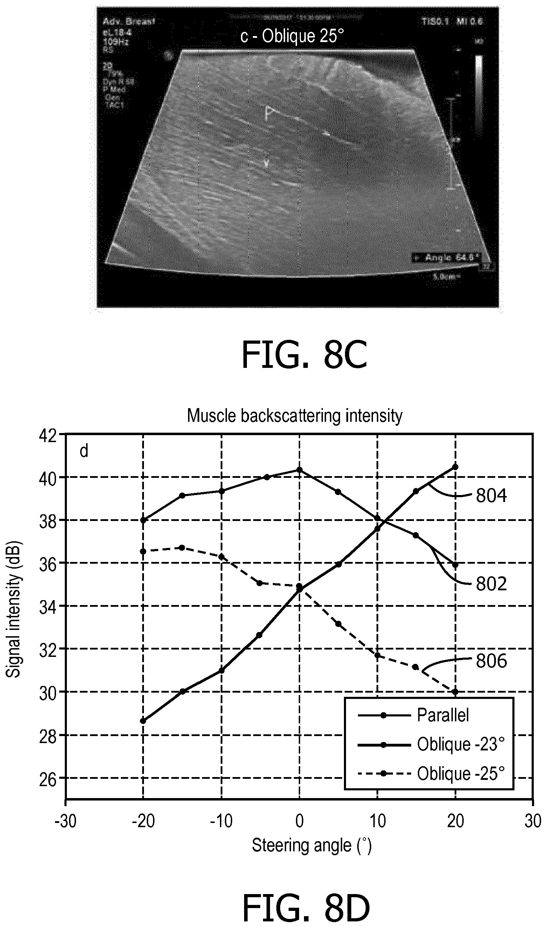

FIGS. 8A-8D show the results of image compounding performed by systems herein, along with a graphical representation of backscattering intensity values versus steering angle for differently oriented tissues. FIG. 8A shows the final angularly compounded image generated by combining the images 602 shown in FIG. 6. One or more processors, e.g., signal processor 225 and/or data processor 227, can be configured to compound the images and produce the image of FIG. 8A, which shows anisotropic tissue oriented parallel to the lateral direction of the imaging plane. FIG. 8B shows the final angularly compounded image generated by combining the images 702 shown in FIG. 7, the image showing anisotropic tissue oriented at an oblique angle of approximately -23° with respect to the lateral direction of the imaging plane. FIG. 8C shows a final angularly compounded image generated by combining a plurality of images arranged at an oblique angle of approximately 25° with respect to the lateral direction of the imaging plane. FIG. 8D is a line plot graphing the measured backscattering signal intensity as a function of the beam steering angle for the three angular orientations of the fiber shown in FIGS. 8A-8C. The line representing parallel tissue alignment 802 reaches a peak intensity at a 0° steering angle, the line representing a -23° tissue alignment 804 reaches a peak at a 20° steering angle, and the line representing a 25° tissue alignment 806 reaches a peak at a -20° steering angle. Accordingly, in-plane fiber orientations can be determined by searching for a global maximum of backscattering signal intensity detected via a plurality of transmit/receive beam steering angles. By determining the peak backscattering signal intensity, systems herein can determine the orientation of the anisotropic tissue fibers with respect to the imaging plane of the transducer used to image the fibers.

After determining the angular orientation of the targeted tissue fibers, systems herein can adjust the transmission angle of the push/tracking beams accordingly, so that push and tracking beams are transmitted at each of a (1) fiber in-plane and parallel to the lateral direction, (2) fiber cross-plane direction, and (3) fiber in-plane with a known tilt angle direction. The beam steering angle can be adjusted in real-time based on the fiber orientation. A display processor, e.g., display processor 228, in cooperation with a user interface, e.g., user interface 230, can display an indication of the automated adjustment of push/tracking beams implemented by the system in a manner similar to that shown in FIGS. 4 and/or 5A. According to the invention, a shape-adjusted ROI tracking box may be displayed on a live ultrasound image. The shape of the tracking box may be adjusted as push/tracking beams are emitted at various angles with respect to the tissue.

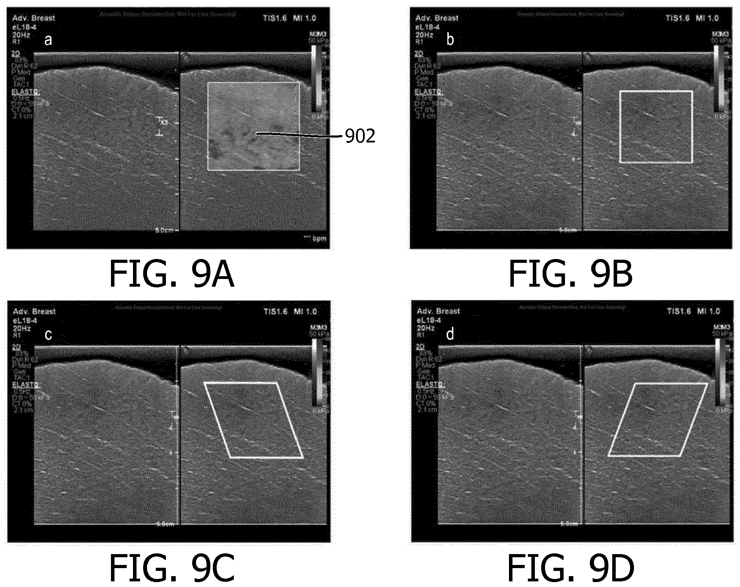

FIGS. 9A-9D show example ultrasound images that may be displayed to a user during transmission of the push/tracking beams into anisotropic tissue arranged at an oblique angle with respect to the lateral direction of the imaging plane. FIG. 9A shows a live tissue stiffness map 902 generated using a conventional push/tracking ROI configuration, i.e., not responsive to the oblique angular orientation of the tissue fibers. FIG. 9B shows a shear wave ROI tracking box 904 that has not been steered. RIG. 9C shows a shear wave tracking box 906 that has been steered to 20°, and FIG. 9D shows a shear wave tracking box that has been steered to -20°. In various embodiments, full characterization of a target tissue oriented at an oblique angle with respect to the lateral direction of an ultrasound transducer imaging plane may require transmitting push pulses and tracking beams at three or more distinct transmission angles with respect to the target tissue, as illustrated in FIGS. 9B-9D. One push/tracking sequence may be transmitted approximately parallel to the target tissue, a second push/tracking sequence may be transmitted at an approximately perpendicular angle with respect to the target tissue, and a third push/tracking sequence may be transmitted at a specified tilt angle with respect to the target tissue. The specified tilt angle is determined based on the angular orientation of the fiber determined either visually or in automated fashion. Each distinct steering angle will generate a shear wave that propagates in a different direction within the target tissue.

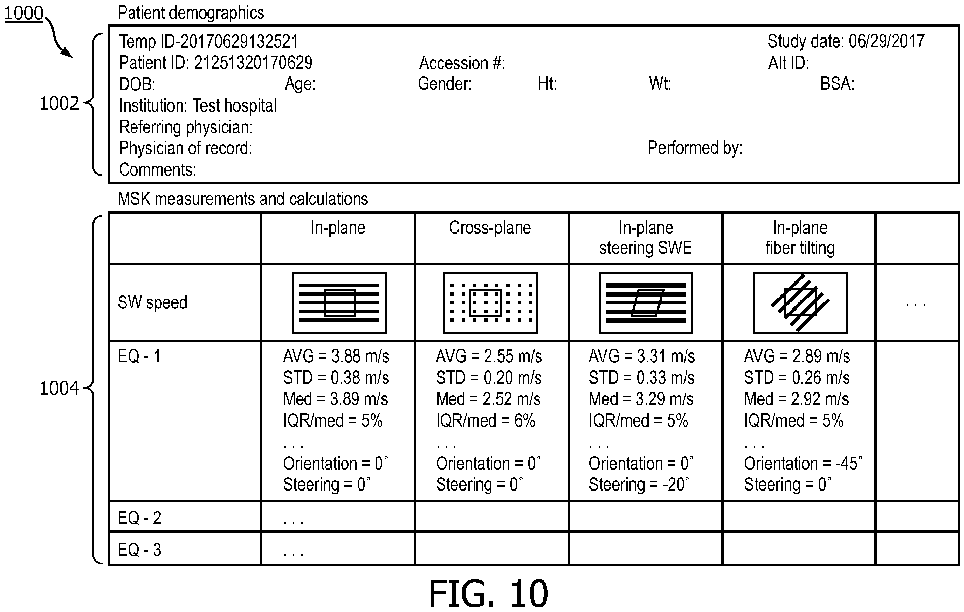

The tissue stiffness map 902 shown in FIG. 9A may also be displayed concurrently with the live ultrasound images shown in each of FIGS. 9B-9C, for example superimposed on top of or adjacent to the ROI tracking box. The stiffness map may be updated in real time as the ultrasound transducer and/or tissue fiber is moved or as differently-steered push/tracking beams are emitted. Systems herein are configured to reconstruct the stiffness map in accordance with the steering angle of the beamformer. In some examples, a user interface may receive a user input, e.g., "Save" or "Stop," instructing the system to measure the absolute stiffness of a sub-region or point within the ROI tracking box, which may also be defined by the user. The shape and size of the subregions may vary. Stiffness measurements can be stored in a memory and displayed in a final report, e.g., report 1000 as shown in FIG. 10, in conjunction with the acquisition conditions employed to acquire the measurements.

Adjustment of the ROI tracking boxes shown in FIGS. 9A-9D (and 5A) represents beam steering performed by multiple system components described herein. For example, the transmit beamformer, e.g., beamformer 221, can be configured transmit one or more push pulses into the targeted tissue at the angle determined by the system. A controller, e.g., beamformer controller 224, can be configured to receive the transmission angle determined by a processor, e.g., data processor 227, and direct the transmit beamformer to emit a push pulse at the designated angle. Tracking beams used to monitor tissue displacement caused by the resulting shear wave can be emitted at the same transmission angle, i.e., parallel to the push pulse. A receive beamformer, e.g., multiline receive beamformer 222, can then receive echoes responsive to the transmitted tracking beams. The tracking beams are aligned with the push pulse direction so that resulting tissue motion is aligned with the acoustic beam axis for optimal motion signal-to-noise ratio.

To accurately determine tissue stiffness values, systems described herein can be configured to process the information received by a data acquisition unit (via the receive beamformer) to reconstruct shear wave speed in a manner that accounts for the steering of the push pulses and tracking beams. Using one or more processors, e.g., data processor 227, the system can be configured to convert ultrasound scanning data of shear wave-induced tissue displacement to scanning data of shear wave-induced tissue displacement measured at a particular angle with respect to the imaging plane of the ultrasound transducer. One or more processors can also be configured to perform shear wave speed vector estimation in both the lateral and axial direction to account for the true propagation angle of the shear wave. Shear wave speed reconstruction can then be performed by combining the lateral and axial velocity components into a true, composite shear wave velocity, thus accounting for the fact that shear wave generated by an angular push pulse will displace tissue in both the axial and lateral direction. In this manner, systems here are configured to reconstruct the shear wave velocity in a manner that is adaptive to the steering angle of the push and tracking pulses used to generate the shear wave. The final shear wave reconstruction can be determined by combining the underlying shear wave propagation pattern, push/tracking steering angle, and the angular orientation of the fiber with respect to the imaging plane of the ultrasound probe.

FIG. 10 is an example report 1000 generated and displayed in accordance with principles of the present disclosure. As shown, the report 1000 may include a variety of patient demographic information 1002 and shear wave tissue characterization information 1004. The characterization information 1004 can be categorized according to the angular orientation of the target tissue and/or the steering angle of the beamformer. For example, the information 1004 can include whether the ultrasound transducer used to perform shear wave imaging transmitted push pulses in-plane, cross-plane, in-plane at a steered angle, or in-plane against obliquely oriented target tissue. The information 1004 may also include, for each steering angle and/or fiber orientation, an average shear wave speed, a shear wave speed standard deviation, a median shear wave speed, and an interquartile range of the shear wave speed. The orientation angle of the fibers comprising the target tissue can be displayed, along with the steering angle of the push pulses/tracking beams. By displaying shear wave information obtained via a variety of fiber orientations and steering angles, multi-parametric stiffness quantification of the target tissue is embodied in the report 1000. This information may improve the accuracy of the tissue stiffness assessment performed by a user, e.g., a radiologist, by revealing differences in shear wave propagation across different fiber orientations. By tabulating the information in the manner shown, the shear wave imaging conditions used to obtain the stiffness quantifications may be repeated upon subsequent examinations, thereby enabling a consistent, standardized tissue interrogation technique.

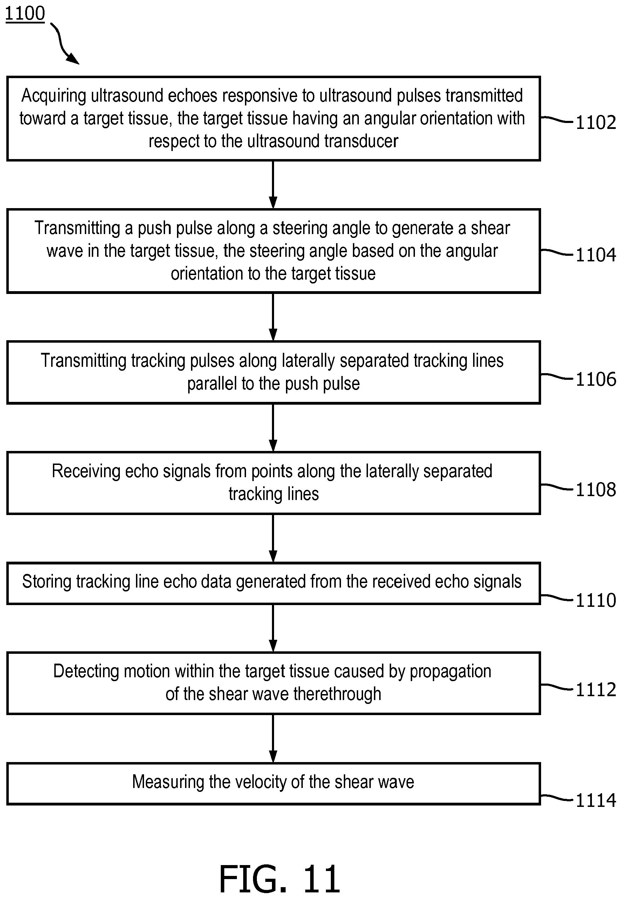

FIG. 11 is a flow diagram of a method of shear wave imaging performed in accordance with principles of the present disclosure. The example method 1100 shows the steps that may be utilized , in any sequence, by the systems and/or apparatuses described herein. The method 1100 may be performed by an ultrasound imaging system, such as system 100, or other systems including, for example, a mobile system such as LUMIFY by Koninklijke Philips N.V. ("Philips"). Additional example systems may include SPARQ and/or EPIQ, also produced by Philips.

In the embodiment shown, the method 1100 begins at block 1102 by "acquiring ultrasound echoes responsive to ultrasound pulses transmitted toward a target tissue, the target tissue having an angular orientation with respect to the ultrasound transducer."

At block 1104, the method involves "transmitting a push pulse along a steering angle to generate a shear wave in the target tissue, the steering angle based on the angular orientation of the target tissue."

At block 1106, the method involves "transmitting tracking pulses along laterally separated tracking lines parallel to the push pulse."

At block 1108, the method involves "receiving echo signals from points along the laterally separated tracking lines."

At block 1110, the method involves "storing tracking line echo data generated from the received echo signals."

At block 1112, the method involves "detecting motion within the target tissue caused by propagation of the shear wave therethrough."

At block 1114, the method involves "measuring the velocity of the shear wave."

In various embodiments where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art as far as they fall within the scope of the present system as set forth in the claims that follow. Accordingly, the specification and drawings are to be regarded in an illustrative manner and are not intended to limit the scope of the appended claims.

**Claims**

1. An ultrasound imaging system (200) for shear wave imaging comprising:

   an ultrasound transducer configured to acquire echoes responsive to ultrasound pulses transmitted toward a target tissue, the target tissue comprising anisotropic tissue having an angular orientation with respect to a nominal axial direction of the ultrasound transducer;
   a beamformer (222) configured to:

   control the ultrasound transducer to transmit a push pulse along a steering angle for generating a shear wave in the target tissue, wherein the steering angle is based on the angular orientation of the target tissue;
   transmit, from the ultrasound transducer, tracking pulses along laterally separated tracking lines parallel to the push pulse; and
   receive, from the ultrasound transducer, echo signals from points along the laterally separated tracking lines; and

   a processor (227) in communication with the beamformer and configured to:

   store tracking line echo data generated from the received echo signals;
   in response to the tracking line echo data, detect motion within the target tissue caused by propagation of the shear wave therethrough; and
   measure the velocity of the shear wave,

**characterized in that** the ultrasound imaging system further comprises a user interface (230) configured to display a live ultrasound image of the target tissue and an ROI tracking box, the ROI tracking box configured to change shape in response to adjustment of the steering angle of the push pulse.

2. The ultrasound imaging system (200) of claim 1, wherein the processor (227) is configured to determine the angular orientation responsive to user input.

3. The ultrasound imaging system (200) of claim 1, wherein the processor (227) is further configured to determine the angular orientation of the target tissue based on the acquired echoes.

4. The ultrasound imaging system (200) of claim 3, wherein the processor (227) is configured to determine the angular orientation of the target tissue by determining an intensity of backscattering signals generated at a plurality of image beam steering angles transmitted by the beamformer (222).

5. The ultrasound imaging system (200) of claim 1, wherein the processor (227) is configured to determine an angular orientation of the target tissue by performing a Hough transform on image frames generated from the acquired echoes.

6. The ultrasound imaging system (200) of any of claims 1 to 5, wherein the processor (227) is configured to detect motion by determining tissue displacement in a lateral and an axial direction caused by the shear wave.

7. The ultrasound imaging system (200) of claim 6, wherein the processor (227) is configured to measure the velocity of the shear wave in the lateral and the axial direction.

8. The ultrasound imaging system (200) of any of claims 1 to 7, wherein the processor (227) is further configured to generate a shear wave map based on the measured velocity of the shear wave, the shear wave map comprising a display of a two dimensional image of shear wave velocity values.

9. The ultrasound imaging system (200) of any of claims 1 to 8, wherein the processor (227) is further configured to determine multiple shear wave velocities obtained at a plurality of push pulse steering angles and angular tissue orientations.

10. The ultrasound imaging system (200) of claim 9, wherein the processor (227) is further configured to determine multi-parametric stiffness values of the target tissue based on the multiple shear wave velocities.

11. The ultrasound imaging system (200) of any of claims 1 to 10, wherein the beamformer (222) is configured to transmit, from the ultrasound transducer, a plurality of push pulses, each push pulse transmitted at a distinct steering angle with respect to the target tissue such that a first push pulse is transmitted parallel to the target tissue, a second push pulse is transmitted perpendicular to the target tissue, and a third push pulse is transmitted at an oblique angle with respect to the target tissue.

12. A method (1100) of shear wave imaging, the method comprising:

acquiring (1102) ultrasound echoes responsive to ultrasound pulses transmitted toward a target tissue, the target tissue comprising anisotropic tissue having an angular orientation with respect to a nominal axial direction of the ultrasound transducer;
transmitting (1104) a push pulse along a steering angle to generate a shear wave in the target tissue, the steering angle based on the angular orientation of the target tissue;
transmitting (1106) tracking pulses along laterally separated tracking lines parallel to the push pulse;
receiving (1108) echo signals from points along the laterally separated tracking lines;
storing (1110) tracking line echo data generated from the received echo signals;
detecting (1112) motion within the target tissue caused by propagation of the shear wave therethrough; and
measuring (1114) the velocity of the shear wave, **characterized in that** the method further comprises:

displaying a live ultrasound image of the target tissue and an ROI tracking box; and
changing the shape of the ROI tracking box in response to adjustment of the steering angle of the push pulse.

13. The method (1100) of claim 12, further comprising determining the angular orientation of the target tissue.

14. The method (1100) of claim 12 or 13, wherein transmitting (1104) a push pulse comprises transmitting a plurality of push pulses, each push pulse transmitted at a distinct steering angle with respect to the target tissue such that a first push pulse is transmitted parallel to the target tissue, a second push pulse is transmitted perpendicular to the target tissue, and a third push pulse is transmitted at an oblique angle with respect to the target tissue.

15. A non-transitory computer-readable medium comprising executable instructions, which when executed cause a processor of an ultrasound imaging system to perform any of the methods of claims 12 to 14.

**Patentansprüche**

1. Ultraschall-Bildgebungssystem (200) für ScherwellenBildgebung, umfassend: einen Ultraschallwandler, der konfiguriert ist, um als Reaktion auf Ultraschallimpulse, die in Richtung eines Zielgewebes gesendet werden, Echos zu erfassen, wobei das Zielgewebe anisotropes Gewebe mit einer Winkelausrichtung in Bezug auf eine nominale axiale Richtung des Ultraschallwandlers umfasst; einen Strahlformer (222), der zu Folgendem konfiguriert ist: steuern des Ultraschallwandlers, um einen Stoßimpuls entlang eines Lenkungswinkels zu übertragen, um eine Scherwelle in dem Zielgewebe zu erzeugen, wobei der Lenkungswinkel auf der Winkelausrichtung des Zielgewebes basiert; senden, von dem Ultraschallwandler, von Verfolgungsimpulsen entlang seitlich getrennter Verfolgungslinien parallel zu dem Stoßimpuls; und empfangen, von dem Ultraschallwandler, von Echosignalen von Punkten entlang der seitlich getrennten Verfolgungslinien; und einen Prozessor (227) in Kommunikation mit dem Strahlformer und der zu Folgendem konfiguriert ist: speichern von Verfolgungslinien-Echosignalen, die von den empfangenen Echosignalen erzeugt werden; als Reaktion auf die Verfolgungslinien-Echodaten, Erfassen einer Bewegung innerhalb des Zielgewebes, die durch Ausbreitung der Scherwelle durch dieses hindurch verursacht wird; und messen der Geschwindigkeit der Scherwelle, **dadurch gekennzeichnet, dass** das Ultraschall-Bildgebungssystem ferner eine Benutzerschnittstelle (230) umfasst, die konfiguriert ist, um ein Live-Ultraschallbild des Zielgewebes und ein ROI-Verfolgungsfeld anzuzeigen, wobei das ROI-Verfolgungsfeld konfiguriert ist, um seine Form als Reaktion auf eine Einstellung des Lenkwinkels des Stoßimpulses zu ändern.

2. Ultraschall-Bildgebungssystem (200) nach Anspruch 1, wobei der Prozessor (227) konfiguriert ist, um die Winkelausrichtung als Reaktion auf eine Benutzereingabe zu bestimmen.

3. Ultraschall-Bildgebungssystem (200) nach Anspruch 1, wobei der Prozessor (227) ferner konfiguriert ist, um die Winkelausrichtung des Zielgewebes basierend auf dem erfassten Echo zu bestimmen.

4. Ultraschall-Bildgebungssystem (200) nach Anspruch 3, wobei der Prozessor (227) konfiguriert ist, um die Winkelausrichtung des Zielgewebes zu bestimmen, indem er eine Intensität von Rückstreusignalen bestimmt, die bei einer Vielzahl von Bildstrahl-Lenkungswinkeln erzeugt werden, die von dem Strahlformer (222) übertragen werden.

5. Ultraschall-Bildgebungssystem (200) nach Anspruch 1, wobei der Prozessor (227) konfiguriert ist, um eine Winkelausrichtung des Zielgewebes zu bestimmen, indem er eine Hough-Transformation an Bildframes durchführt, die aus den erfassten Echos erzeugt werden.

6. Ultraschall-Bildgebungssystem (200) nach einem der Ansprüche 1 bis 5, wobei der Prozessor (227) konfiguriert ist, um eine Bewegung zu erfassen, indem er eine Gewebeverschiebung in einer lateralen und einer axialen Richtung bestimmt, die durch die Scherwelle verursacht wird.

7. Ultraschall-Bildgebungssystem (200) nach Anspruch 6, wobei der Prozessor (227) konfiguriert ist, um die Geschwindigkeit der Scherwelle in der lateralen und der axialen Richtung zu messen.

8. Ultraschall-Bildgebungssystem (200) nach einem der Ansprüche 1 bis 7, wobei der Prozessor (227) ferner konfiguriert ist, um eine Scherwellenkarte basierend auf der gemessenen Geschwindigkeit der Scherwelle zu erzeugen, wobei die Scherwellenkarte eine Anzeige eines zweidimensionalen Bilds von Scherwellengeschwindigkeitswerten umfasst.

9. Ultraschall-Bildgebungssystem (200) nach einem der Ansprüche 1 bis 8, wobei der Prozessor (227) ferner konfiguriert ist, um mehrere Scherwellengeschwindigkeiten zu bestimmen, die bei einer Vielzahl von Stoßimpuls-Lenkungswinkeln und Winkelausrichtungen des Gewebes erlangt werden.

10. Ultraschall-Bildgebungssystem (200) nach Anspruch 9, wobei der Prozessor (227) ferner konfiguriert ist, um multiparametrische Steifigkeitswerte des Zielgewebes basierend auf den mehrfachen Scherwellengeschwindigkeiten zu bestimmen.

11. Ultraschall-Bildgebungssystem (200) nach einem der Ansprüche 1 bis 10, wobei der Strahlformer (222) konfiguriert ist, um von dem Ultraschallwandler eine Vielzahl von Stoßimpulsen zu übertragen, wobei jeder Stoßimpuls in einem bestimmten Lenkungswinkel in Bezug auf das Zielgewebe übertragen wird, sodass ein erster Stoßimpuls parallel zu dem Zielgewebe übertragen wird, ein zweiter Stoßimpuls senkrecht zu dem Zielgewebe übertragen wird und ein dritter Stoßimpuls in einem schrägen Winkel in Bezug auf das Zielgewebe übertragen wird.

12. Verfahren (1100) zur Scherwellenbildgebung, das Verfahren umfassend: erfassen (1102) von Ultraschallechos, die als Reaktion auf Ultraschallimpulse, die in Richtung eines Zielgewebes gesendet werden, wobei das Zielgewebe anisotropes Gewebe mit einer Winkelausrichtung in Bezug auf eine nominale axiale Richtung des Ultraschallwandlers umfasst; senden (1104) eines Stoßimpulses entlang eines Lenkwinkels, um eine Scherwelle in dem Zielgewebe zu erzeugen, wobei der Lenkwinkel auf der Winkelausrichtung des Zielgewebes basiert; senden (1106) von Verfolgungsimpulsen entlang seitlich getrennter Verfolgungslinien parallel zu dem Druckimpuls; empfangen (1108) von Echosignalen von Punkten entlang der seitlich getrennten Verfolgungslinien; speichern (1110) der aus den empfangenen Echosignalen erzeugten Verfolgungslinien-Echodaten; erfassen (1112) einer Bewegung innerhalb des Zielgewebes, die durch Ausbreitung der Scherwelle durch dieses hindurch verursacht wird; und messen (1114) der Geschwindigkeit der Scherwelle, **dadurch gekennzeichnet, dass** das Verfahren ferner Folgendes umfasst: anzeigen eines Live-Ultraschallbilds des Zielgewebes und eines ROI-Verfolgungsfelds; und ändern der Form des ROI-Verfolgungsfelds als Reaktion auf eine Einstellung des Lenkwinkels des Stoßimpulses.

13. Verfahren (1100) nach Anspruch 12, ferner umfassend ein Bestimmen der Winkelausrichtung des Zielgewebes.

14. Verfahren (1100) nach Anspruch 12 oder 13, wobei das Übertragen (1104) eines Stoßimpulses das Übertragen einer Vielzahl von Stoßimpulsen umfasst, wobei jeder Stoßimpuls in einem bestimmten Lenkungswinkel in Bezug auf das Zielgewebe übertragen wird, sodass ein erster Stoßimpuls parallel zu dem Zielgewebe übertragen wird, ein zweiter Stoßimpuls senkrecht zu dem Zielgewebe übertragen wird und ein dritter Stoßimpuls in einem schrägen Winkel in Bezug auf das Zielgewebe übertragen wird.

15. Nicht-transitorisches computerlesbares Medium, umfassend ausführbare Anweisungen, die, wenn sie ausgeführt werden, einen Prozessor eines Ultraschall-Bildgebungssystems veranlassen, eines der Verfahren der Ansprüche 12 bis 14 durchzuführen.

**Revendications**

1. Système d'imagerie ultrasonore (200) pour l'imagerie par ondes de cisaillement comprenant: un transducteur à ultrasons configuré pour acquérir des échos en réponse à des impulsions ultrasonores transmises vers un tissu cible, le tissu cible comprenant un tissu anisotrope ayant une orientation angulaire par rapport à une direction axiale nominale du transducteur à ultrasons; un formateur de faisceau (222) configuré pour: commander le transducteur à ultrasons pour transmettre une impulsion de poussée le long d'un angle de direction pour générer une onde de cisaillement dans le tissu cible, l'angle de direction étant basé sur l'orientation angulaire du tissu cible; transmettre, à partir du transducteur à ultrasons, des impulsions de suivi le long de lignes de suivi séparées latéralement et parallèles à l'impulsion de poussée; et recevoir, du transducteur à ultrasons, des signaux d'écho provenant de points le long des lignes de suivi latéralement séparées; et un processeur (227) en communication avec le formateur de faisceau et configuré pour: stocker des données d'écho de ligne de suivi générées à partir des signaux d'écho reçus; en réponse aux données d'écho de ligne de suivi, détecter un mouvement à l'intérieur du tissu cible provoqué par la propagation de l'onde de cisaillement à travers celui-ci; et mesurer la vitesse de l'onde de cisaillement, **caractérisé en ce que** le système d'imagerie ultrasonore comprend en outre une interface utilisateur (230) configurée pour afficher une image échographique en direct du tissu cible et une boîte de suivi de ROI, la boîte de suivi de ROI étant configurée pour changer de forme en réponse à l'ajustement de l'angle de direction du pousser le pouls.

2. Système d'imagerie ultrasonore (200) selon la revendication 1, dans lequel le processeur (227) est configuré pour déterminer l'orientation angulaire en réponse à une entrée de l'utilisateur.

3. Système d'imagerie ultrasonore (200) selon la revendication 1, dans lequel le processeur (227) est en outre configuré pour déterminer l'orientation angulaire du tissu cible sur la base des échos acquis.

4. Système d'imagerie ultrasonore (200) selon la revendication 3, dans lequel le processeur (227) est configuré pour déterminer l'orientation angulaire du tissu cible en déterminant une intensité de signaux de rétrodiffusion générés à une pluralité d'angles d'orientation du faisceau d'image transmis par le formeur de faisceau. (222).

5. Système d'imagerie ultrasonore (200) selon la revendication 1, dans lequel le processeur (227) est configuré pour déterminer une orientation angulaire du tissu cible en effectuant une transformée de Hough sur les trames d'images générées à partir des échos acquis.

6. Système d'imagerie ultrasonore (200) selon l'une quelconque des revendications 1 à 5, dans lequel le processeur (227) est configuré pour détecter un mouvement en déterminant le déplacement du tissu dans une direction latérale et axiale provoqué par l'onde de cisaillement.

7. Système d'imagerie ultrasonore (200) selon la revendication 6, dans lequel le processeur (227) est configuré pour mesurer la vitesse de l'onde de cisaillement dans la direction latérale et axiale.

8. Système d'imagerie ultrasonore (200) selon l'une quelconque des revendications 1 à 7, dans lequel le processeur (227) est en outre configuré pour générer une carte d'onde de cisaillement basée sur la vitesse mesurée de l'onde de cisaillement, la carte d'onde de cisaillement comprenant un affichage d'une image bidimensionnelle des valeurs de vitesse des ondes de cisaillement.

9. Système d'imagerie ultrasonore (200) selon l'une quelconque des revendications 1 à 8, dans lequel le processeur (227) est en outre configuré pour déterminer de multiples vitesses d'ondes de cisaillement obtenues à une pluralité d'angles de direction d'impulsions de poussée et d'orientations angulaires des tissus.

10. Système d'imagerie ultrasonore (200) selon la revendication 9, dans lequel le processeur (227) est en outre configuré pour déterminer des valeurs de rigidité multiparamétriques du tissu cible sur la base des multiples vitesses d'ondes de cisaillement.

11. Système d'imagerie ultrasonore (200) selon l'une quelconque des revendications 1 à 10, dans lequel le formateur de faisceau (222) est configuré pour transmettre, à partir du transducteur ultrasonore, une pluralité d'impulsions de poussée, chaque impulsion de poussée étant transmise selon un angle de direction distinct par rapport à au tissu cible de telle sorte qu'une première impulsion de poussée soit transmise parallèlement au tissu cible, une deuxième impulsion de poussée soit transmise perpendiculairement au tissu cible, et une troisième impulsion de poussée soit transmise selon un angle oblique par rapport au tissu cible.

12. Procédé (1100) d'imagerie par ondes de cisaillement, le procédé comprenant: acquérir (1102) des échos ultrasonores en réponse à des impulsions ultrasonores transmises vers un tissu cible, le tissu cible comprenant un tissu anisotrope ayant une orientation angulaire par rapport à une direction axiale nominale du transducteur ultrasonore; transmettre (1104) une impulsion de poussée le long d'un angle de direction pour générer une onde de cisaillement dans le tissu cible, l'angle de direction étant basé sur l'orientation angulaire du tissu cible; transmettre (1106) des impulsions de suivi le long de lignes de suivi séparées latéralement et parallèles à l'impulsion de poussée; recevoir (1108) des signaux d'écho provenant de points le long des lignes de poursuite séparées latéralement; stocker (1110) des données d'écho de ligne de suivi générées à partir des signaux d'écho reçus; détecter (1112) un mouvement à l'intérieur du tissu cible provoqué par la propagation de l'onde de cisaillement à travers celui-ci; et mesurer (1114) la vitesse de l'onde de cisaillement, **caractérisé en ce que** le procédé comprend en outre: afficher une image échographique en direct du tissu cible et une boîte de suivi de ROI; et modifier la forme de la boîte de suivi de ROI en réponse à l'ajustement de l'angle de braquage de l'impulsion de poussée.

13. Procédé (1100) selon la revendication 12, comprenant en outre la détermination de l'orientation angulaire du tissu cible.

14. Procédé (1100) selon la revendication 12 ou 13, dans lequel la transmission (1104) d'une impulsion de poussée comprend la transmission d'une pluralité d'impulsions de poussée, chaque impulsion de poussée étant transmise selon un angle de direction distinct par rapport au tissu cible de telle sorte qu'une première impulsion de poussée est transmise parallèlement au tissu cible, une deuxième impulsion de poussée est transmise perpendiculairement au tissu cible, et une troisième impulsion de poussée est transmise selon un angle oblique par rapport au tissu cible.

15. Support non transitoire lisible par ordinateur comprenant des instructions exécutables qui, lorsqu'elles sont exécutées, amènent un processeur d'un système d'imagerie par ultrasons à exécuter l'un quelconque des procédés des revendications 12 à 14.

FIG. 1

FIG. 2

EP 3 749 210 B1

302  303

FIG. 3A

EP 3 749 210 B1

FIG. 3B

FIG. 3C

FIG. 4

EP 3 749 210 B1

FIG. 5A

FIG. 5B

FIG. 6

FIG. 7

FIG. 8A

FIG. 8B

FIG. 8C

FIG. 8D

FIG. 9A

FIG. 9B

FIG. 9C

FIG. 9D

Patient demographics

EP 3 749 210 B1

1000
1002

| | | | | |
|---|---|---|---|---|
| Temp ID-20170629132521 | | | | Study date: 06/29/2017 |
| Patient ID: 21251320170629 | Accession #: | | | Alt ID: |
| DOB: Age: | Gender: | Ht: | Wt: | BSA: |
| Institution: Test hospital | | | | |
| Referring physician: | | | | |
| Physician of record: | | | Performed by: | |
| Comments: | | | | |

MSK measurements and calculations

1004

| | In-plane | Cross-plane | In-plane steering SWE | In-plane fiber tilting | |
|---|---|---|---|---|---|
| SW speed | | | | | . . . |
| EQ - 1 | AVG = 3.88 m/s STD = 0.38 m/s Med = 3.89 m/s IQR/med = 5% . . . Orientation = 0° Steering = 0° | AVG = 2.55 m/s STD = 0.20 m/s Med = 2.52 m/s IQR/med = 6% . . . Orientation = 0° Steering = 0° | AVG = 3.31 m/s STD = 0.33 m/s Med = 3.29 m/s IQR/med = 5% . . . Orientation = 0° Steering = -20° | AVG = 2.89 m/s STD = 0.26 m/s Med = 2.92 m/s IQR/med = 5% . . . Orientation = -45° Steering = 0° | |
| EQ - 2 | . . . | | | | |
| EQ - 3 | . . . | | | | |

FIG. 10

<u>1100</u>

```
┌─────────────────────────────────────────────────────────┐
│ Acquiring ultrasound echoes responsive to ultrasound     │
│ pulses transmitted toward a target tissue, the target    │──1102
│ tissue having an angular orientation with respect to the │
│ ultrasound transducer                                     │
└─────────────────────────────────────────────────────────┘
```

Acquiring ultrasound echoes responsive to ultrasound pulses transmitted toward a target tissue, the target tissue having an angular orientation with respect to the ultrasound transducer ——1102

Transmitting a push pulse along a steering angle to generate a shear wave in the target tissue, the steering angle based on the angular orientation to the target tissue ——1104

Transmitting tracking pulses along laterally separated tracking lines parallel to the push pulse ——1106

Receiving echo signals from points along the laterally separated tracking lines ——1108

Storing tracking line echo data generated from the received echo signals ——1110

Detecting motion within the target tissue caused by propagation of the shear wave therethrough ——1112

Measuring the velocity of the shear wave ——1114

# FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2018000455 A1
- US 20130131511, Peterson

### Non-patent literature cited in the description

- **J. L. GENNISSON et al.** Viscoelastic and Anisotropic Mechanical Properties of in vivo Muscle Tissue Assessed by Supersonic Shear Imaging. *Ultrasound in Medicine and Biology, New York, NY, US,* 01 May 2010, vol. 36 (5), ISSN 0301-5629, 789-801
- Mapping Myocardial Fiber Orientation Using Echocardiography-Based Shear Wave Imaging. **W.-N. LEE et al.** IEEE Transactions on Medical Imaging. IEEE Service Center, 01 March 2012, vol. 31, 554-562
- Imaging Transverse Isotropic Properties of Muscle by Monitoring Acoustic Radiation Force Induced Shear Waves Using a 2-D Matrix Ultrasound Array. **M. WANG et al.** IEEE Transactions on Medical Imaging. IEEE Service Center, 01 September 2013, vol. 32, 1671-1684
- **S. AUBRY et al.** Viscoelasti city in Achilles Tendonopathy: Quantitative Assessment by Using Real-time Shear-Wave Elastography. *Radiology,* 01 March 2015, vol. 274 (3), ISSN 0033-8419, 821-829